(19) Europäisches Patentamt
European Patent Office
Office européen des brevets

(11) Publication number: **0 101 641 A2**

(12) **EUROPEAN PATENT APPLICATION**

(21) Application number: **83304156.9**

(22) Date of filing: **18.07.83**

(51) Int. Cl.³: **C 07 D 451/04**, A 61 K 31/46 // C07D451/14, C07D498/06, C07D513/06

(30) Priority: **22.07.82 GB 8221163**

(43) Date of publication of application: **29.02.84 Bulletin 84/9**

(84) Designated Contracting States: **CH DE FR GB IT LI NL**

(71) Applicant: **BEECHAM GROUP PLC, Beecham House Great West Road, Brentford Middlesex (GB)**

(72) Inventor: **Wootton, Gordon, 51 Parkway, Sawbridgeworth Herts (GB)**

(74) Representative: **Stott, Michael John et al, European Patent Attorney Beecham Pharmaceuticals Great Burgh Yew Tree Bottom Road, Epsom Surrey KT18 5XQ (GB)**

(54) **Sulfonylamino-aza-bicyclo-alkyl derivatives.**

(57) A compound of formula (I), or pharmaceutically acceptable salt, or N-oxide thereof, or a solvate of any of the foregoing:

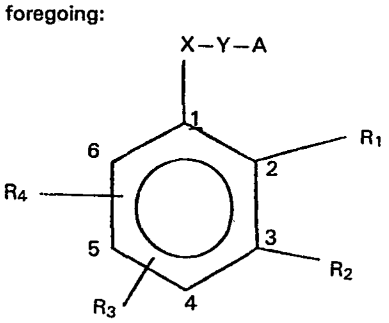

(I)

wherein:

one of X and Y is CO and the other is $NR_9$, $R_9$ being hydrogen or as defined with $R_1$;

A is a group

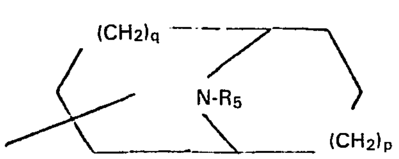

(II)

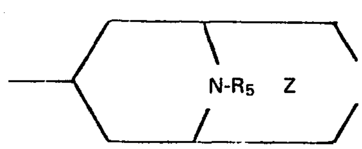

(III)

or

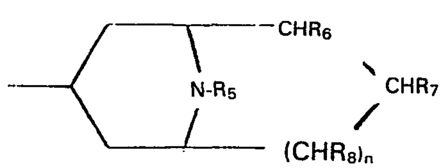

(IV)

where

p and q each independently are 0 to 2;

Z is O or S;

n is 0 or 1; and

one of $R_6$ and $R_7$ when n = 0 is $C_{1-4}$ alkoxy, $C_{1-4}$ alkoxycarbonyl, hydroxy of $C_{1-4}$ alkyl optionally substituted by hydroxy, $C_{1-4}$ alkoxy or $C_{1-4}$ acyloxy, and the other is hydrogen or $C_{1-4}$ alkyl or one of $R_6$, $R_7$ and $R_8$ when n = 1 is $C_{1-4}$ alkyl and the other two are the same or different and are hydrogen or $C_{1-4}$ alkyl.

$R_5$ is $C_{1-7}$ alkyl, $-(CH_2)_sR_{10}$, s being 0 to and $R_{10}$ being $C_{3-8}$

EP 0 101 641 A2

cycloalkyl, $-(CH_2)_tR_{11}$, t being 1 or 2 and $R_{11}$ being thienyl or phenyl optionally substituted by one or two substituents selected from $C_{1-4}$ alkoxy, trifluoromethyl, halogen, nitro, carboxy, esterified carboxy and $C_{1-4}$ alkyl optionally substituted by hydroxy, $C_{1-4}$ alkoxy, carboxy, esterified carboxy or in vivo hydrolysable acyloxy:

and either

$R_1$ is $C_{1-6}$ alkoxy, $C_{1-6}$ alkylthio or, together with $R_9$ when Y is $NR_9$, $C_{1-2}$ alkylene,

one of $R_2$, $R_3$ and $R_4$ is $R_{15}SO_2NR_{20}$ where $R_{15}$ is $C_{1-6}$ alkyl, or amino optionally substituted by one or two groups selected from $C_{1-6}$ alkyl, $C_{3-8}$ cycloalkyl, $C_{3-8}$ cycloalkyl $C_{1-4}$ alkyl, phenyl or phenyl $C_{1-4}$ alkyl groups, any of which phenyl groups may be substituted by one or more groups selected from halogen, trifluoromethyl, $C_{1-6}$ alkyl, $C_{1-6}$ alkoxy and nitro, or optionally substituted by $C_{4-5}$ polymethylene and $R_{20}$ is hydrogen or $C_{1-4}$ alkyl, and the other two of $R_2$, $R_3$ and $R_4$ together are $C_{1-2}$ alkylenedioxy, or the other two are the same or different and are selected from hydrogen, halogen, trifluoromethyl, $C_{1-6}$ alkyl, $C_{1-6}$ alkoxy, $C_{1-7}$ acyl, carboxylic $C_{1-7}$ acylamino, $C_{1-6}$ alkylsulphonyl, $C_{1-6}$ alkylsulphinyl, hydroxy, nitro or amino, aminocarbonyl or aminosulphonyl optionally N-substituted by one or two $C_{1-6}$ alkyl, $C_{3-8}$ cycloalkyl, $C_{3-8}$ cycloalkyl $C_{1-4}$ alkyl groups or optionally N-disubstituted by $C_{4-5}$ polymethylene,

or

$R_1$ and $R_2$ together are $C_{1-2}$ alkylendioxy;

one of $R_3$ and $R_4$ is $R_{15}SO_2NR_{20}$ as defined, and the other is selcted from the groups defined hereinbefore for $R_3$ and $R_4$.

0101641

PTD/B1300

TITLE MODIFIED
see front page

NOVEL COMPOUNDS

This invention relates to novel compounds having useful pharmacological properties, in particular dopamine antagonist activity, to pharmaceutical compositions containing them, and to a process for their preparation.

European Patent Application No. 79302978.6 discloses that compounds of the formula (A), and pharmaceutically acceptable salts thereof:

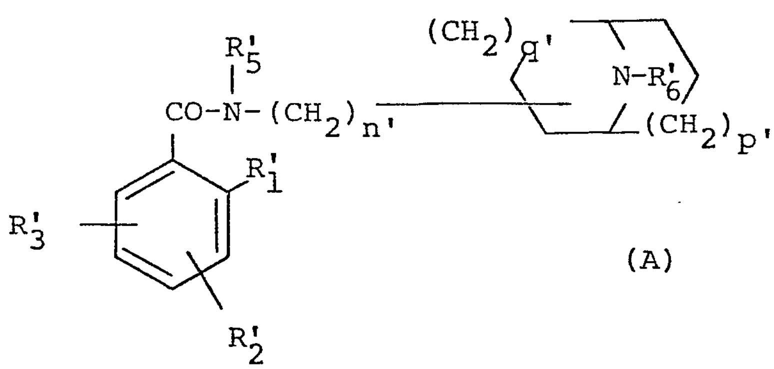

(A)

wherein:

$R_1'$ is a $C_{1-6}$ alkoxy group;

$R_2'$ and $R_3'$ are the same or different and are hydrogen, halogen, $CF_3$, $C_{2-7}$ acyl, $C_{2-7}$ acylamino, or amino, aminocarbonyl or aminosulphone optionally substituted by one or two $C_{1-6}$ alkyl groups, $C_{1-6}$ alkylsulphone or nitro;

$R_5'$ is hydrogen or $C_{1-6}$ alkyl;

$R_6'$ is $C_{1-7}$ alkyl or a group $-(CH_2)_sR_7'$ where s is 0 to 2 and $R_7'$ is a $C_{3-8}$ cycloalkyl group, or a group $-(CH_2)_{t'}R_8'$ where t' is 1 or 2 and $R_8'$ is $C_{2-5}$ alkenyl or a phenyl group optionally substituted by one or two substituents selected from $C_{1-6}$ alkyl, $C_{1-4}$ alkoxy, trifluoromethyl and halogen; and

n', p' and q' are independently 0 to 2; have useful pharmacological activity. More specifically the compounds of formula (A) are stated to be useful in the treatment of disorders related to impaired gastro-intestinal motility and/or in the treatment of disorders of the central nervous system. All the compounds are stated to have anti-emetic activity.

U.K. Patent Application No. 2078715A discloses compounds of the formula (B):

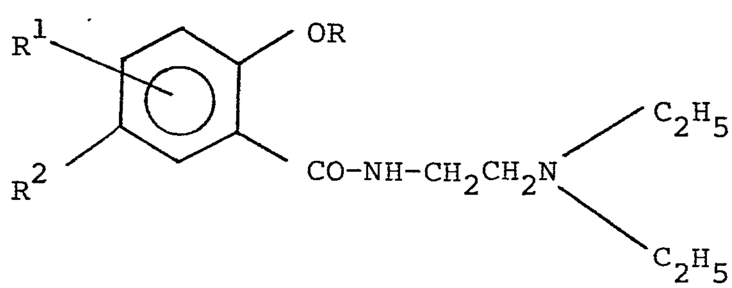

(B)

wherein R is alkyl;

$R^1$ is hydrogen, halogen, alkyl or alkoxy; and

$R^2$ is amino, alkanoyl or

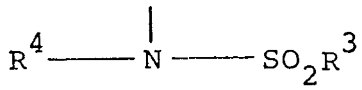

$R^3$ being alkyl or dialkylamino and
$R^4$ being hydrogen or alkyl;
with the proviso that when $R^2$ is amino or alkanoyl, $R^1$ is alkyl; and pharmaceutically acceptable or veterinarily acceptable acid addition salts thereof. These are useful as gastric disorder remedies or as antiemetics.

A structurally distinct group of compounds has now been discovered, which compounds have dopamine antagonist activity.

Accordingly, the present invention provides a compound of formula (I), or a pharmaceutically acceptable salt, or N-oxide thereof, or a solvent adduct of any of the foregoing.

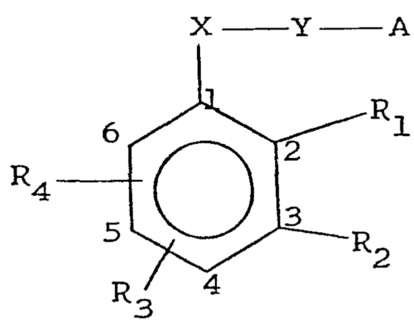

(I)

wherein:

one of X and Y is CO and the other is $NR_9$, $R_9$ being hydrogen or, with $R_1$, as defined below, when X is CO and Y is $NR_9$

A is a group

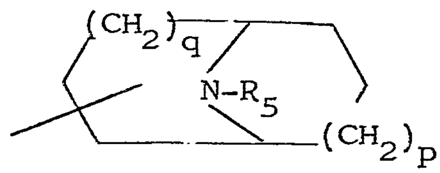

(II)

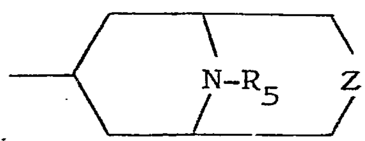

(III)

or

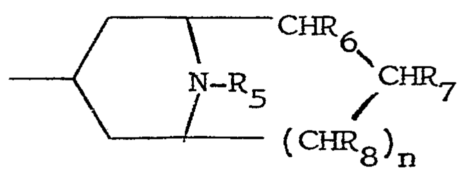

(IV)

where

p and q each independently are 0 to 2;

Z is O or S;

n is 0 or 1; and

one of $R_6$ and $R_7$ when n=0 is $C_{1-4}$ alkoxy, $C_{1-4}$ alkoxycarbonyl, hydroxy or $C_{1-4}$ alkyl optionally substituted by hydroxy, $C_{1-4}$ alkoxy or $C_{1-4}$ acyloxy, and the other is hydrogen or $C_{1-4}$ alkyl or one of $R_6$, $R_7$ and $R_8$ when n=1 is $C_{1-4}$ alkyl and the other two are the same or different and are hydrogen or $C_{1-4}$alkyl.

$R_5$ is $C_{1-7}$ alkyl, $-(CH_2)_sR_{10}$, s being 0 to 2 and $R_{10}$ being $C_{3-8}$ cycloalkyl, $-(CH_2)_tR_{11}$, t being 1 or 2 and $R_{11}$ being thienyl or phenyl optionally substituted by one or two substituents selected from $C_{1-4}$ alkoxy, trifluoromethyl, halogen, nitro, carboxy, esterified carboxy and $C_{1-4}$ alkyl optionally substituted by hydroxy, $C_{1-4}$ alkoxy, carboxy, esterified carboxy or in vivo hydrolysable acyloxy:

and either

$R_1$ is $C_{1-6}$ alkoxy, $C_{1-6}$ alkylthio or, together with $R_9$ when Y is $NR_9$, $C_{1-2}$ alkylene,

one of $R_2$, $R_3$ and $R_4$ is $R_{15}SO_2NR_{20}$ where $R_{15}$ is $C_{1-6}$ alkyl, or amino optionally substituted by one or two groups selected from $C_{1-6}$ alkyl, $C_{3-8}$ cycloalkyl, $C_{3-8}$ cycloalkyl $C_{1-4}$ alkyl, phenyl or phenyl $C_{1-4}$ alkyl groups, any of which phenyl groups may be substituted by one or more groups selected from halogen, trifluoromethyl, $C_{1-6}$ alkyl, $C_{1-6}$ alkoxy and nitro, or optionally substituted by $C_{4-5}$ polymethylene and $R_{20}$ is hydrogen or $C_{1-4}$alkyl, and the other two together are $C_{1-2}$ alkylenedioxy, or the other two are the same or different and are selected from hydrogen, halogen, trifluoromethyl, $C_{1-6}$ alkyl, $C_{1-6}$ alkoxy, $C_{1-6}$ alkylthio, $C_{1-7}$ acyl, carboxylic $C_{1-7}$ acylamino, $C_{1-6}$ alkylsulphonyl, $C_{1-6}$ alkylsulphinyl, hydroxy, nitro or amino, aminocarbonyl or aminosulphonyl optionally N-substituted by one or two groups selected from $C_{1-6}$ alkyl, $C_{3-8}$ cycloalkyl, $C_{3-8}$ cycloalkyl $C_{1-4}$ alkyl, phenyl or phenyl $C_{1-4}$alkyl groups or optionally N-disubstituted by $C_{4-5}$ polymethylene,

or

$R_1$ and $R_2$ together are $C_{1-2}$ alkylenedioxy;

one of $R_3$ and $R_4$ is $R_{15}SO_2NR_{20}$ as defined, and the other is selected from the groups defined hereinbefore for $R_3$ and $R_4$.

Preferably, X is CO and Y is $NR_9$, $R_9$ preferably being hydrogen or, as defined with $R_1$, $C_{1-2}$ alkylene.

A preferred value of A is of formula (II).

When A is a group of formula (II) as defined, p is suitably 0 or 1, preferably 1, and q is suitably 0 or 1.

Often the group Y and the heterobicycle nitrogen atom are separated by 2 or 3 carbon atoms preferably 3.

The X.Y. moiety is preferably in an equatorial orientation to the heterobicycle ring.

When A is a group of formula (III) as defined Z is O or S, preferably O.

When A is a group of formula (IV) as defined, preferably each of $R_6$ and $R_7$ when n=0 and each of $R_6$, $R_7$ and $R_8$ when n=1 are in the exo-position.

Preferred examples for one of $R_6$ and $R_7$ when n=0 are methoxy, ethoxy, n-propoxy, methyl, ethyl, n-propyl, methoxycarbonyl and ethoxycarbonyl, hydroxy, hydroxymethyl or hydroxyethyl, methoxymethyl, methoxyethyl, ethoxymethyl, ethoxyethyl, acetylmethyl and acetylethyl. The most preferred is methyl.

Preferred examples for one of $R_6$, $R_7$ and $R_8$ when n=1 are methyl, ethyl and n-propyl.

Preferred examples for the other of $R_6$ and $R_7$ when n=0 and for the other two of $R_6$, $R_7$ and $R_8$ when n=1 are hydrogen, methyl, ethyl and n- and iso-propyl.

Examples of $R_5$ when $C_{1-7}$ alkyl include methyl, ethyl and n-and isopropyl. Within $C_{1-7}$ alkyl, $C_{4-7}$ alkyl are of interest, especially those of the form $(CH_2)_uR_{14}$ wherein u is 1 or 2 and $R_{14}$ is a secondary or tertiary $C_{3-6}$ alkyl group. Examples of $C_{4-7}$ alkyl include n-sec- and tert-butyl, n-pentyl, n-hexyl and n-heptyl, and especially iso-butyl, 3-methylbutyl, 2,2-dimethylpropyl and 3,3-dimethylbutyl.

Preferred examples of $R_5$, when $-(CH_2)_s R_{10}$ are those wherein s is 1 or 2, in particular those wherein $R_{10}$ is $C_{5-8}$ cycloalkyl, such as cyclohexyl, and cyclopropyl.

Preferred examples of $R_5$, when $-(CH_2)_tR_{11}$, are those wherein t is 1. $R_{11}$ may be 2- or 3-thienyl or preferably is phenyl optionally substituted by one of $C_{1-4}$ alkoxy, trifluoromethyl, halogen, carboxy, esterified carboxy or $C_{1-4}$ alkyl optionally substituted by hydroxy, $C_{1-4}$ alkoxy, carboxy, esterified carboxy and *in vivo* hydrolysable acyloxy.

When phenyl is substituted by optionally substituted $C_{1-4}$ alkyl, examples of $C_{1-4}$ alkyl include methyl, ethyl, n- and iso-propyl, and n-, iso-, sec- and tert- butyl; methyl however is preferred. Examples of substituents of such alkyl groups include hydroxy, methoxy, ethoxy, n- and iso - propoxy, carboxy, esterified carboxy, and *in vivo* hydrolysable acyloxy. The substitution preferably occurs on the terminal carbon atom of the alkyl group.

Examples of esterified carboxy groups include $C_{1-4}$ alkoxycarbonyl, such as methoxy-, ethoxy-, n- and iso-propoxy-carbonyl, phenoxycarbonyl or benzyloxycarbonyl, either being optionally substituted in the phenyl ring by one or two substituents selected from $C_{1-4}$ alkyl, $C_{1-4}$ alkoxy, trifluoromethyl, halogen or nitro.

Examples of *in vivo* hydrolysable acyloxy groups include $C_{1-6}$ alkanoyloxy, for example acetoxy, propionoxy, *n*- and *iso*- butyroxy, and 2,3 dimethylpropanyloxy, benzyloxy or benzenesulphonyloxy either being optionally substituted in the phenyl ring by one or two substituents selected from $C_{1-4}$ alkyl, $C_{1-4}$ alkoxy, trifluoromethyl, halogen or nitro, or other sulphonyloxy groups, for example $C_{1-6}$ alkanesulphonyloxy group, such as methanesulphonyloxy.

The most preferred examples of $R_5$, when $-(CH_2)_tR_{11}$, are those wherein t is 1 and $R_{11}$ is unsubstituted phenyl or monosubstituted phenyl in particular mono-p-substituted phenyl. Examples of preferred p-substituents include methyl, trifluoromethyl, fluoro, chloro and bromo, especially fluoro. Unsubstituted benzyl, *p*-fluorobenzyl, *p*-chlorobenzyl and *p*-methylbenzyl are especially preferred examples of $R_5$.

When $R_1$ is $C_{1-6}$ alkoxy, $C_{1-6}$ alkylthio or together with $R_9$ when Y is $NR_9$, $C_{1-2}$ alkylene, preferred examples of $R_1$ include methoxy, ethoxy, *n*- and *iso*-propoxy, methylthio, ethylthio, *n*- and *iso*-propylthio, and, with $R_9$, ethylene and methylene and, with $R_2$, ethylenedioxy and methylenedioxy. Most preferably, $R_1$ is methoxy.

One of $R_2$, $R_3$ and $R_4$ is then $R_{15}SO_2NR_{20}$ as defined.

Preferably $R_{20}$ is hydrogen.

Examples of $R_{15}C_{1-6}$ alkyl groups include methyl, ethyl and n- and iso-propyl. $R_{15}$ is favourably methyl or ethyl, in particular methyl.

Other examples of $R_{15}$ groups include amino and amino substituted by one or two methyl, ethyl, n- or iso-propyl, n-, sec- or tert-butyl cylopropyl, cyclobutyl, cyclopentyl, cyclohexyl, cycloheptyl; cyclopropylmethyl, phenyl, cyclobutylmethyl, cyclopentylmethyl, cyclohexylmethyl, or benzyl groups or substituted by $C_4$ or $C_5$ polymethylene.

Preferred of such $R_{15}$ groups include methylamino and dimethylamino.

The other two of $R_2$, $R_3$ and $R_4$ are together methylenedioxy or ethylenedioxy or may suitably be the same or different and are hydrogen, chloro, bromo, methoxy, ethoxy, n- or iso-propoxy, methylthio, ethylthio, methyl, ethyl, methylsulphonyl, ethylsulphonyl or nitro. Most preferably, the other two of $R_2$, $R_3$ and $R_4$ are hydrogen, chloro, bromo, methyl or methylsulphonyl.

When $R_1$ and $R_2$ together are $C_{1-2}$ alkylenedioxy, one of $R_3$ and $R_4$ is then $R_{15}SO_2NR_{20}$ as defined.

Suitable and preferred $R_3$ or $R_4$ $R_{15}SO_2NR_{20}$ are as so described hereinbefore.

The other of $R_3$ and $R_4$ may then suitably be hydrogen, chloro, bromo, methoxy, ethoxy, n- or iso-propoxy, methylthio, ethylthio, methyl, ethyl, methylsulphonyl, ethylsulphonyl, or nitro. Most preferably $R_3$ and $R_4$ are each independently hydrogen, chloro, bromo, methyl or methylsulphonyl.

$R_3$ is preferably in the 4-position with respect to X as the 1-position; $R_4$ is preferably in the 5-position similarly defined.

Salts of the compounds of the formula (I) include acid addition salts and quaternary salts. Salification at the bicycle 8-nitrogen atom only is of interest in the present invention.

Suitable acid addition salts of the compound of formula (I) include pharmaceutically acceptable inorganic salts such as the sulphate, nitrate, phosphate, borate, hydrochloride and hydrobromide and pharmaceutically acceptable organic acid addition salts such as acetate, tartrate, maleate, citrate, succinate, benzoate, ascorbate, methanesulphonate, α-ketoglutarate, α-glycerophosphate,

Examples of salts also include such compounds quaternised by compounds such as $R_{19}$- E wherein $R_{19}$ is $C_{1-6}$ alkyl, phenyl-$C_{1-6}$ alkyl or $C_{5-7}$ cycloalkyl, and E is a radical corresponding to an anion of an acid. Suitable examples of $R_{19}$ include methyl, ethyl and n- and iso-propyl; and benzyl and phenethyl. Suitable E include halide such as chloride, bromide and iodide.

The compounds of formula (I) may also form pharmaceutically acceptable N-oxides.

The compounds of the formula (I) and their pharmaceutically acceptable salts and N-oxides may also form solvates.

BAD ORIGINAL

It will of course be realised that the compounds of the formula (I) have chiral or prochiral centres, and thus are capable of existing in a number of stereoisomeric forms, including enantiomers. The invention extends to each of these stereoisomeric forms, and to mixtures thereof (including racemates). The different stereoisomeric forms may be separated or resolved one from the other by the usual methods, or any given isomer may be obtained by stereospecific or asymmetric synthesis.

Preferred compounds of formula (I) include those containing the moiety of formula (V):

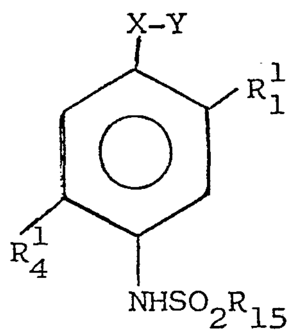

(V)

wherein X, Y and $R_{15}$

$R_1^1$ is $C_{1-6}$ alkoxy; and

$R_4^1$ is hydrogen, chloro or $C_{1-6}$ alkyl sulphonyl.

Suitable and preferred $R_4^1$ are as so described for corresponding $R_4$ under formula (I).

A preferred group of compounds within those of formula (I), is of formula (VI):

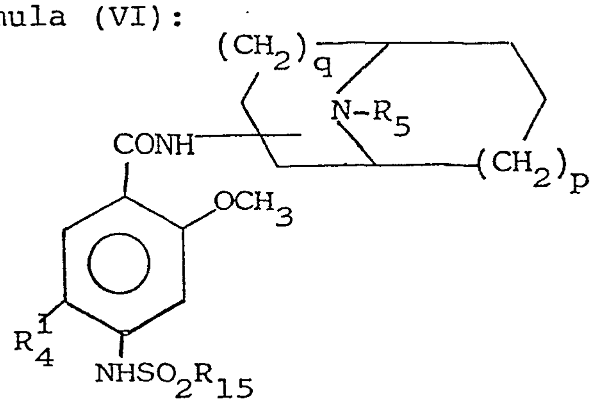

(VI)

wherein the variables are as defined in formulae (I), (II) and (V).

Suitable and preferred $R_4^1$ are as so described under formula (V). Suitable and preferred remaining variables are as so described under formula (I).

More suitably p is 0 or 1, it is believed preferably 0. Preferably q is 1 and the CONH moiety is then attached at the 3-position (conventional numbering) and in the β orientation).

A sub-group of compounds within those of formula (VI) are those of the formula (VII):

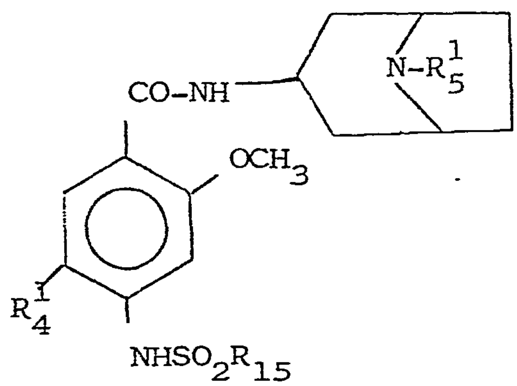

(VII)

wherein

$R_5^1$ is $C_{4-7}$ alkyl or $-(CH_2)_sR_{10}$ where s is 0 to 2 and $R_{10}$ is $C_{5-8}$ cycloalkyl; and the remaining variables are as defined in formula (V).

Suitable and preferred $R_{15}$ are as so described under formula (I).

Suitable and preferred $R_5^1$ are as so described under formula (I) for corresponding $R_5$.

Suitable and preferred $R_4^1$ are as so described under formula (V).

A group of compounds within those of formula (VII) is that wherein $R_5^1$ is $(CH_2)_uR_{14}$ as defined or $(CH_2)_uR_{10}$ where s, u, $R_{10}$ and $R_{16}$ are as defined.

Examples of $R_5^1$ in these compounds include iso-butyl, 3-methylbutyl, 2,2-dimethylpropyl, 3,3-dimethylbutyl and cyclopropylethyl.

It is preferred that the CONH moiety is in the β-orientation to the nortropane ring.

A sub-group of compounds within those of formula (VI) is of the formula (VIII):

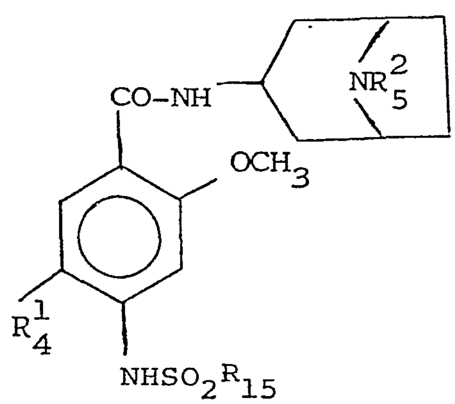

(VIII)

wherein

$R_5^2$ is thienylmethyl or $-(CH_2)_tR_{11}$, t being 1 or 2, and $R_{11}$ being phenyl optionally substituted by one or two substituents selected from $C_{1-4}$ alkoxy, trifluoromethyl, halogen, carboxy, esterified carboxy, and $C_{1-4}$ alkyl optionally substituted by hydroxy, $C_{1-4}$ alkoxy, carboxy, esterified carboxy or <u>in vivo</u> hydrolysable acyloxy, and the remaining variables are as defined in formula (V).

Particularly preferred compounds are those wherein $R_5^2$ is benzyl optionally substituted in the phenyl ring by one or two substituents selected from $C_{1-4}$ alkoxy, trifluoromethyl, halogen and $C_{1-4}$ alkyl.

It is especially preferred that the phenyl ring is unsubstituted.

Suitable and preferred $R_{15}$ are as so described under formula (I).

Suitable and preferred $R_4^1$ are as so described under formula (V).

It is preferred that the CONH moiety is in the β-orientation to the nortropane moiety.

A third sub-group of compounds within those of formula (VI) is of formula (IX):

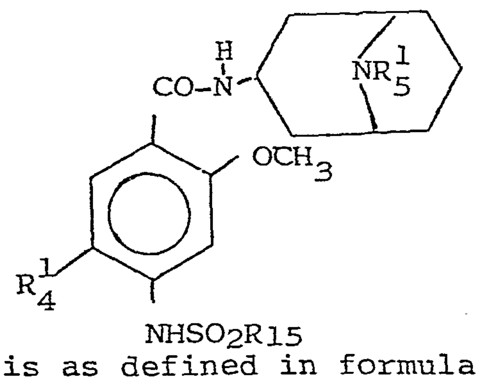

(IX)

wherein $R_5^1$ is as defined in formula (VII), and the remaining variables are as defined in formula (VI).

Suitable and preferred variables are as so described under formula (VII).

Another sub-group of compounds within those of the formula (VI) of interest are those of the formula (X):

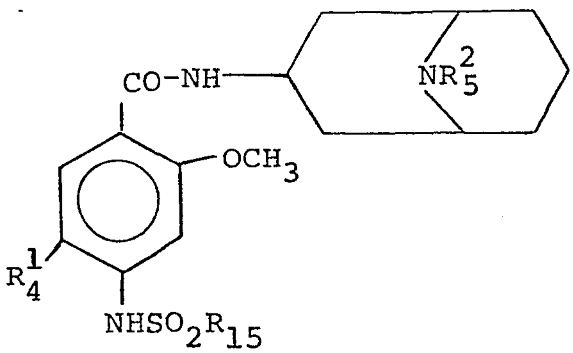

(X)

wherein the variables are as defined in formula (VIII).

Preferred compounds are those wherein $R_5^2$ is optionally substituted benzyl as defined under formula (VII).

It is especially preferred that the phenyl ring in $R_5^2$ is mono-substituted and/or that the substitution is in the para-position and/or that the substituent is chloro, fluoro or methyl.

Suitable and preferred $R^1_4$ and $R_{15}$ are as so described under formula (VIII).

A second group of compounds within those of formula (I) is of formula (XI):

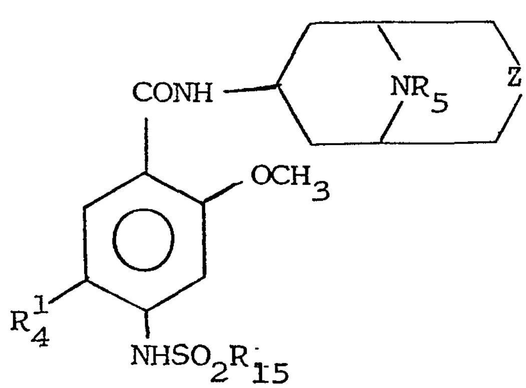

(XI)

wherein the variables are as defined in formulae (I), (III) and (V).

Suitable and preferred variables are as so described under formula (VI).

Preferably Z is O, and the CONH moiety is in the β-orientation.

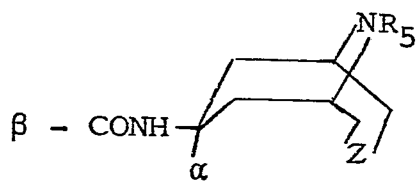

A sub-group of compounds within those of formula (XI) are those of the formula (XII):

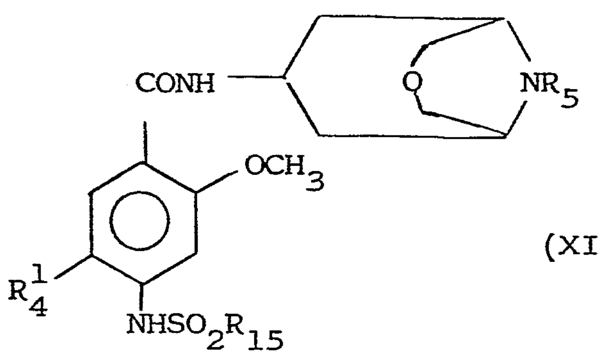

(XII)

wherein the variables are as defined in formula (XI).

Suitable and preferred variables are as so described under formula (XI).

It is preferred that the CONH moiety is in the β-orientation to the oxagranatane ring.

Another sub-group of compounds within those of the formula (XI) of interest are those of the formula (XIII):

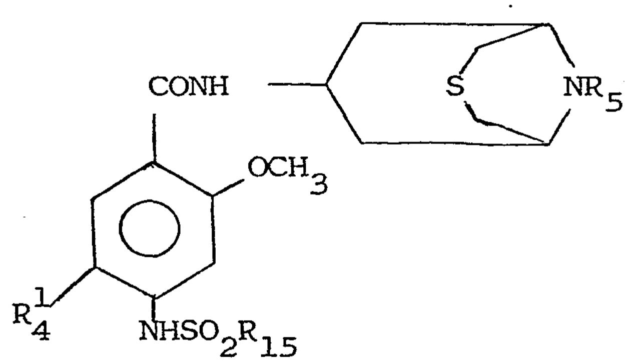

(VIII)

wherein the variables are as defined in formula (XI).

Suitable and preferred variables are as so described under formula (XI).

It is preferred that the CONH moiety is in the β-orientation to the thiagranatane ring.

Within formula (I) is a third group of compounds of formula (XIV):

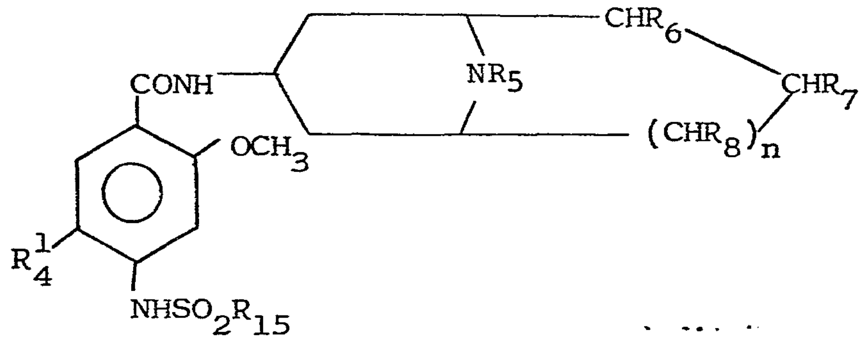

(XIV)

wherein the variables are as defined in formulae (I) and (IV) and (V).

Suitable and preferred $R_4^1$ are as so described under formula (V).

Suitable and preferred remaining variables are as so described under formula (I).

It is preferred that the CONH moiety is in the β-orientation to the bicyclic moiety.

Within formula (XIV) is a sub-group of compounds of formula (XV):

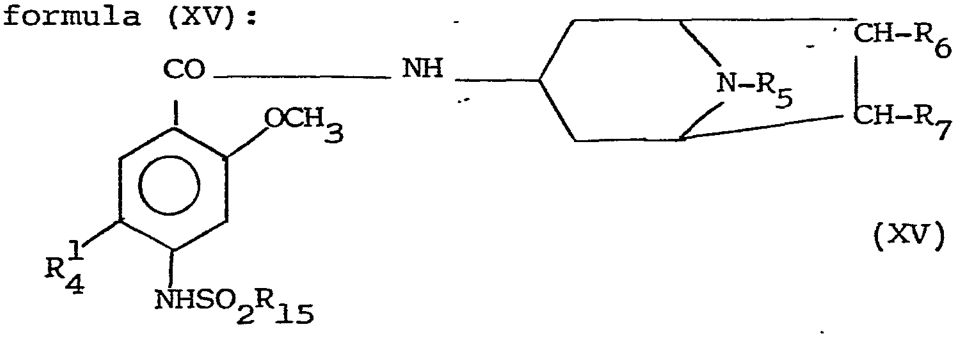

(XV)

wherein the variables are as defined in formula (XIV).

One of $R_6$ and $R_7$ is often methoxy, ethoxy, methyl, ethyl, methoxycarbonyl, ethoxycarbonyl, hydroxy, hydroxymethyl, hydroxyethyl, methoxymethyl, methoxyethyl, ethoxymethyl, ethoxyethyl, acetylmethyl or acetylethyl, and the other is hydrogen, methyl or ethyl.

Preferred $R_5$ are preferred $R_5^2$ as so described under formula (VIII).

Suitable and preferred remaining variables are as so described under formula (XIV).

A second sub-group of compounds within formula (XIV) is of formula (XVI):

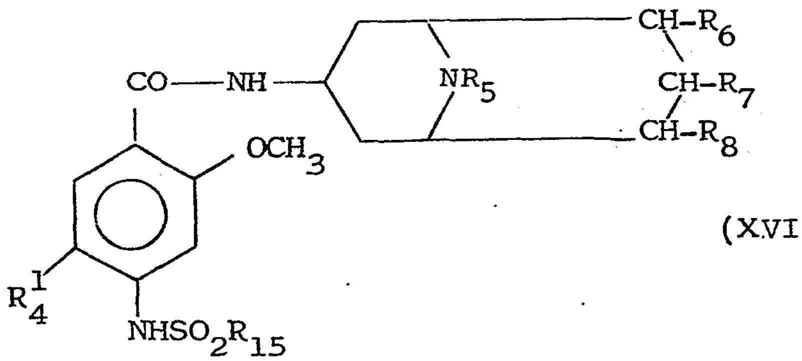

(XVI)

wherein the variables are as defined in formula (XIV).

One of $R_6$, $R_7$ and $R_8$ is often methyl or ethyl and the other two are the same or different and are methyl, ethyl or hydrogen.

Preferred $R_5$ are preferred $R_5^2$ as so described under formula (X).

Suitable and preferred remaining variables are as so described under formula (XIV).

A further group of compounds of formula (I) is of formula (XVII):

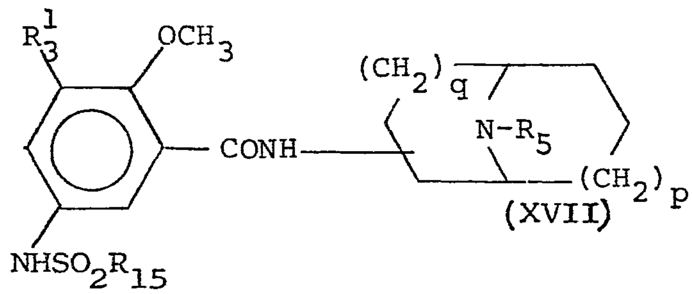

(XVII)

wherein $R_3^1$ is hydrogen or halogen and the remaining variables are as defined in formulae (I) and (II).

Suitable and preferred variables are so described under formula (I) for corresponding variables in formula (I).

The invention also provides a process for the preparation of a compound of the formula (I), which process comprises reacting a compound of the formula (XVIII):

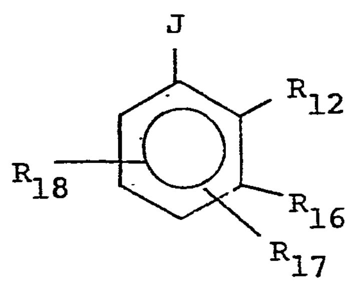

(XVIII)

with a compound of formula (XIX):

$$L{-}A \qquad (XIX)$$

wherein:

one of J and L is $COQ_1$, where $Q_1$ is a leaving group, and the other is $-NH_2$; and

either

$R_{12}$ is $C_{1-6}$ alkoxy or $C_{1-6}$ alkylthio or $(CH_2)_vCR_{13}R_{14}Q_2$ wherein v is 0 or 1, $R_{13}$ and $R_{14}$ are both H or together form an oxo group and $Q_2$ is a leaving group, or $Q_1$ and $Q_2$ are together a bridging oxygen atom;

one of $R_{16}$, $R_{17}$ and $R_{18}$ is $R_{15}SO_2NR_{20}$ as defined, amino $C_{1-6}$ alkylamino or $Q_3-SO_2NR_{20}$ where $Q_3$ is a leaving group; the other two are as defined for $R_2$, $R_3$ and $R_4$ in formula (I) or any groups convertible thereto;

or

$R_{12}$ and $R_{16}$ together are $C_{1-2}$ alkylenedioxy; one of $R_{17}$ and $R_{18}$ is $R_{15}SO_2NR_{20}$ as defined, amino, $C_{1-6}$ alkylamino or $Q_3-SO_2NR_{20}$ as defined; and the other is as defined for $R_2$, $R_3$ or $R_4$ in formula (I), or any groups convertible thereto;

and

A is as defined in formula (I);

with the proviso that when J is amino, $R_2$, $R_3$ or $R_4$ is other than amino, and when $R_{12}$ is $(CH_2)vCR_{13}R_{14}Q_2$, J is $COQ_1$;

and thereafter, when one of $R_{16}$, $R_{17}$ and $R_{18}$ is amino, $C_{1-6}$ alkylamino or $Q_3SO_2NR_{20}$ and not $R_{15}SO_2NR_{20}$ as defined, converting said group to $R_{15}SO_2NR_{20}$; when $R_{12}$ is $(CH_2)_vC\ R_{13}R_{14}Q_2$ and $R_{13}$ and $R_{14}$ form an oxo group, reducing the resulting compound and optionally converting $R_2$, $R_3$ or $R_4$ to another $R_2$, $R_3$ or $R_4$ respectively; as necessary convering $R_5$ to other $R_5$; and optionally forming a pharmaceutically acceptable salt or N-oxide of the resultant compound of the formula (I).

The leaving groups $Q_1$ and $Q_2$ are groups that are readily displaceable by a nucleophile. Examples of such groups are hydroxy, halogen such as chloro and bromo, for $Q_1$ and $Q_2$, when $R_{13}$ and $R_{14}$ together are oxo, acyloxy such as $C_{1-4}$alkanoyloxy, $C_{1-4}$alkoxycarbonyloxy and activated hydrocarbyloxy such as pentachlorophenoxy, and, for $Q_2$ when $R_{13}$ and $R_{14}$ are each hydrogen, labile acyloxy such as tosyloxy, mesyloxy or triflate.

If a leaving group is hydroxy, then the reaction is preferably carried out in an inert non-hydroxylic solvent, such as benzene, toluene or diethyl ether in the presence of a dehydrating catalyst, such as a carbodiimide, for example dicyclohexylcarbodiimide. The reaction may be carried out at a non-extreme temperature such as -10 to 100°C, for example 0 to 80°C.

If a leaving group is a halide or $Q_2$ labile acyloxy, then the reaction is preferably carried out at a non-extreme temperature in an inert non-hydroxylic solvent, such as benzene, toluene or diethyl ether. It is also preferably carried out in the presence of an acid acceptor, such as an organic b ase, in particular a tertiary amine, such as triethylamine, trimethylamine, pyridine or picoline, some of which can also function as the solvent. Alternatively,

the acid acceptor can be inorganic, such as calcium carbonate, sodium carbonate or potassium carbonate.

If a leaving group is acyloxy then the reaction is preferably carried in substantially the same manner as if the leaving group were hydroxy. Suitable examples of acyloxy leaving groups include $C_{1-4}$ alkanoyloxy.

If a leaving group is $C_{1-4}$ alkoxycarbonyloxy, then the reaction is preferably carried out in an inert solvent, such as methylene chloride, at a non-extreme temperature in the presence of an acid acceptor such as triethylamine.

If a leaving group is activated hydrocarbyloxy, then the reaction is preferably carried out in an inert polar solvent, such as dimethylformamide. It is also preferred that the activated hydrocarbyloxy group is a pentachlorophenyl ester and that the reaction is carried out at ambient temperature.

Preferably $Q_1$, when $R_{12}$ is $C_{1-6}$ alkoxy or $C_{1-6}$ alkylthio, is halogen such as chloro.

When $R_{13}$ and $R_{14}$ are both H atoms, $Q_1$ and $Q_2$ are the same or different and preferably are halogen or hydroxy, or $Q_2$ is mesyloxy, tosyloxy or triflate. $Q_1$ may also be $C_{1-4}$ alkanoyloxy or activated hydrocarbyloxy. Conveniently, $Q_1$ and $Q_2$ are the same, in particular the same halogen.

When $R_{13}$ and $R_{14}$ together form an oxo group, $Q_2$ is preferably an activated hydrocarbyloxy group. Alternatively, $Q_1$ and $Q_2$ together are 0, thus forming a derivative of phtalic anhydride (when n = 0) or of homophthalic anhydride (when n = 1).

The conversion of $R_{16}$, $R_{17}$ or $R_{18}$ amino to $R_{15}SO_2NR_{20}$ as defined is effected conventionally using the corresponding compound $R_{15}SO_2Q_4$ where $Q_4$ is a leaving group, such as halide, for example chloride and bromide, or hydroxy. When halide is the leaving group, the reaction is generally carried out in the presence of a base. When hydroxy is the leaving group, the reaction is generally carried out in the presence of a dehydrating agent, such as dicyclohexylcarbodiimide, in an inert solvent at non-extreme temperature, such as ambient temperature or slightly above, or reflux temperature.

Depending on steric and/or electronic factors and/or reaction conditions mono- or di-acylation may occur. If diacylation takes place, subsequent selective monodeacylation of the resultant compound is necessary to form the desired $R_{15}SO_2NR_{20}$ group.

Treatment with a base such as methanolic sodium hydroxide at ambient or slightly elevated temperatures is apt.

It is often most convenient to effect the coupling of compounds (XVIII) and (XIX) using a compound of formula (XVIII) wherein one of $R_{16}$, $R_{17}$ and $R_{18}$ is amino and not $R_{15}SO_2NH$ as defined, and to convert the relevant amino group in the coupling product to $R_{15}SO_2NR_{20}$.

The invention therefore also provides a second process for the preparation of a compound of formula (I), which process comprises reacting a compound of the formula (XX):

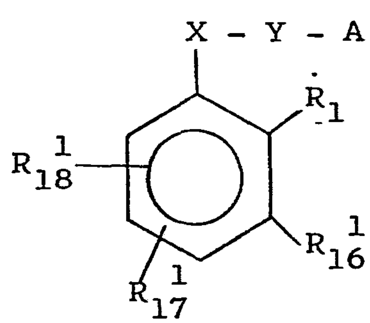

(XX)

wherein $R^1_{16}$, $R^1_{17}$ and $R^1_{18}$ are $R_{16}$, $R_{17}$ and $R_{18}$ as defined but none is $R_{15}SO_2RN_{20}$ or $Q_3SO_2NR_{20}$ as defined, with a compound of formula (XXI):

$$R_{15}SO_2Q_4 \qquad (XXI)$$

as hereinbefore defined; and as necessary thereafter selectively monodeacylating the resultant compound.

Reaction conditions are as described for the corresponding step of the first process hereinbefore.

The conversion of $R_{16}$, $R_{17}$ or $R_{18}$ $Q_3SO_2NR_{20}$ to $R_{15}SO_2NR_{20}$ where $R_{15}$ contains an optionally substituted amino group as defined is effected conventionally using a corresponding amine, or ammonia.

$Q_3$ is a leaving group displaceable by a nucleophile. Suitable $Q_3$ include hydroxy, halogen such as chloro or bromo and labile acyloxy, such as tosyloxy, mesyloxy or triflate.

Reaction conditions are as for corresponding $Q_2$ hereinbefore.

Where $R_{13}$ and $R_{14}$ together form an oxo group, the reduction of the carbonyl group in the thus formed compound is preferably carried out, with or without isolation of the compound, by reduction with tin/hydrochloric acid at a non-extreme temperature.

The skilled man will appreciate that the choice or necessity of conversion of groups $R_{16}$, $R_{17}$ and $R_{18}$ including $R_2$, $R_3$ and $R_4$ to (other) groups $R_2$, $R_3$ and/or $R_4$ will be dictated by the nature and position of substituents $R_1$, $R_2$, $R_3$ and $R_4$.

It will be apparent that compounds of the formula (I) containing an $R_2$, $R_3$, $R_4$ or $R_5$ group which is convertible to another $R_2$, $R_3$, $R_4$ and $R_5$ group are useful novel intermediates. A number of such conversions is possible not only for the end compounds of formula (I), but also for their intermediates as follows:

(a) an hydrogen substituent is convertible to a nitro substituent by nitration;

(b) a nitro substituent is convertible to an amino substituent by reduction;

(c) a $C_{1-4}$ acylamino substituent is convertible to an amino substituent by deacylation;

(d) an amino substiuent is convertible to a $C_{1-4}$ acylamino substituent by acylation;

(e) a hydrogen substituent is convertible to a halogen substituent by halogenation;

(f) a $C_{1-6}$ alkylthio or $C_{1-6}$ alkylsulphinyl substituent is convertible to a $C_{1-6}$ alkylsulphinyl or a $C_{1-6}$ alkylsulphonyl substituent respectively by oxidation.

(g) an $H_2NSO_2NR_{20}$ substituent is convertible to a corresponding substituent substituted by one or two $C_{1-6}$ alkyl, $C_{3-8}$ cycloalkyl, $C_{3-8}$ cycloalkyl $C_{1-4}$ alkyl or phenyl $C_{1-4}$ alkyl groups any of which phenyl groups may be substituted by one or more groups selected from halogen, trifluoromethyl, $C_{1-6}$ alkyl, $C_{1-6}$ alkoxy and nitro, or substituted by $C_{4-5}$ polymethylene by N-alkylation.

Conversions (a) to (g), are only exemplary and are not exhaustive of the possibilities.

In regard to (a), nitration is carried out in accordance with known procedures.

In regard to (b), the reduction is carried out with a reagent suitable for reducing nitroanisole to aminoanisole.

In regard to (c), deacylation is carried out by treatment with a base, such as an alkali metal hydroxide.

In regard to (d), the acylation is carried out with an acylating agent, such as the corresponding acid or acid chloride. Formylation is carried out with the free acid.

In regard to (e), halogenation is carried out with conventional halogenating agents.

In regard to (f), oxidation is carried out at below ambient temperatures in a non-aqueous solvent, such as a chlorinated hydrocarbon, in the presence of an organic peracid, such as 3-chloroperbenzoic acid, or in water in the presence of a soluble strong inorganic oxidant, such as an alkali metal permanganate or in aqueous hydrogen peroxide. It will be realised that this process may also N-oxidise the N-$R_5$ moiety and suitable precautions will routinely be taken by the skilled man.

In regard to (g), alkylation is carried out with a corresponding alkylating agent such as the chloride or bromide under conventional conditions.

$R_5$ optionally substituted benzyl as hereinbefore defined may be replaced by other $R_5$. Such $R_5$ benzyl groups may for example be removed, when $R_2$, $R_3$ or $R_4$ is not halogen by conventional transition metal catalysed hydrogenolysis to give compounds of the formula (XXII):

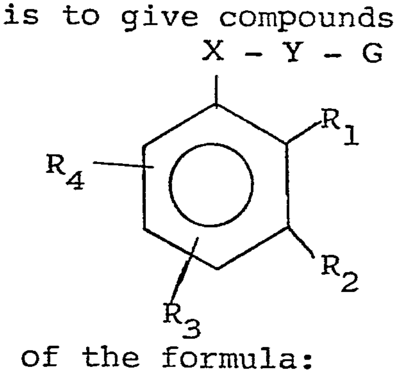

(XXII)

wherein G is of the formula:

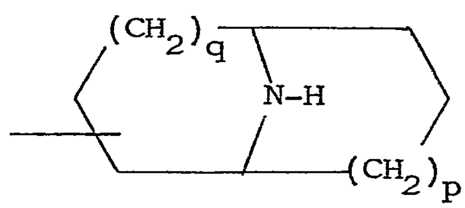

(XXIII)

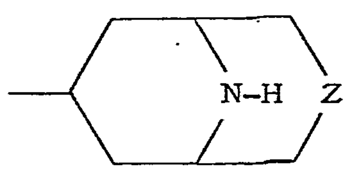

(XXIV)

or

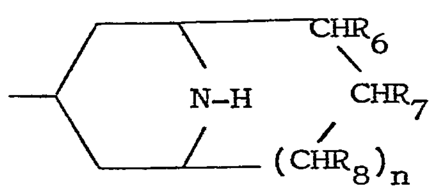

(XXV)

and the variable groups are as defined in formula (I).

This invention also provides third process for the preparation of a compound of the formula (I) which comprises the reaction of a corresponding compound of the formula (XXII) as hereinbefore defined with a compound $Q_5R_5$ wherein $R_5$ is as defined in formula (I) and $Q_5$ is a leaving group, and optionally forming a pharmaceutically acceptable salt or N-oxide of the resulting compound of the formula (I).

Suitable values for $Q_5$ include groups readily displaced by nucleophiles such as Cl, Br, I, $OSO_2CH_3$ or $OSO_2C_6H_4pCH_3$.

Favoured values for $Q_5$ include Cl, Br and I.

The reaction may be carried out under conventional alkylation conditions for example in an inert solvent such as dimethylformamide in the presence of an acid acceptor such as potassium carbonate. Generally the reaction is carried out at non-extreme temperature such as at ambient or slightly above.

Inter-converting $R_5$ in the compound of the formula (XVIII) before coupling with the compound of the formula (XIX) is preferred. Such interconversions are effected conveniently under the above conditions. It is desirable to protect any amine function with a group readily removable by acidolysis such as a $C_{2-7}$ alkanoyl group before $R_5$ interconversion.

The substituents in the phenyl ring when $R_5$ is benzyl in a compound of formula (I), in particular the substituted $C_{1-4}$ alkyl substituents, are interconvertible. A number of such interconversions are possible not only for the end

BAD ORIGINAL

compounds of formula (I), but also for their intermediates as follows:

(i) a carboxy $C_{1-4}$alkyl substituent is convertible to an esterified carboxy $C_{1-4}$ alkyl substituent by esterification:

(ii) an esterified carboxy $C_{1-4}$ alkyl substituent is convertible to a carboxy $C_{1-4}$ alkyl substituent by deesterification;

(iii) $C_{1-4}$ alkoxy $C_{1-4}$ alkyl substituent or an in vivo hydrolysable $C_{2-4}$ acyloxy $C_{1-4}$ alkyl substituent is convertible to an hydroxy $C_{1-4}$ alkyl substituent by deetherification or deesterification:

(iv) an optionally esterified carboxy or carboxy $C_{1-3}$ alkyl substituent is convertible to an hydroxymethyl or hydroxy $C_{2-4}$ alkyl substituent by reduction; and

(v) a hydroxy $C_{1-4}$ alkyl is convertible to $C_{1-4}$ alkoxy $C_{1-4}$alkyl by O-alkylation or to in vivo hydrolysable $C_{2-4}$ acyloxy $C_{1-4}$ alkyl by O-acylation.

Conversions (i) to (iv) are only exemplary and are not exhaustive of the possibilities.

In regard to (i) and (ii), the esterification and ed-esterification reactions are carried out in conventional manner.

In regard to (iii), a $C_{1-4}$ alkoxy $C_{1-4}$ alkyl substituent is convertible to an hydroxy $C_{1-4}$ alkyl substituent by conventional methods, such as warming with aqueous hydrobromic acid or by treatment with pyridine hydrochloride, boron tribromide, boron triodide or iodotrimethylsilane.

An *in vivo* hydrolysable $C_{2-4}$ acyloxy $C_{1-4}$ alkyl substituent is convertible to an hydroxy $C_{1-4}$ alkyl substituent by acid or base hydrolysis.

In regard to (iv), the reduction is carried out with a selective metal complex hydride, for example lithium aluminium hydride, under conventional conditions.

In regard to (v), O-alkylation is carried out under conventional conditions in an inert solvent at a non-extreme temperature such as ambient temperature or slightly above or at reflux temperature. The $C_{1-4}$ alkylating agent has a leaving group that is readily displaceable by a nucleophile. Examples of leaving groups include halide, such as chloride, bromide or iodide, or labile acyloxy groups, such as mesyl and tosyl.

O-acylation is carried out under conventional conditions with an acylating agent which has an acyl group capable of forming an *in vivo* hydrolysable acyloxy group and a leaving group, such as halide, for example chloride and bromide, and hydrogen. When halide is the leaving group, the reaction is generally carried out in the presence of a base. When hydroxy is the leaving group, the reaction is generally carried out in the presence of a dehydrating agent, such as dicyclohexylcarbodiimide, in an inert solvent at non-extreme temperature, such as ambient temperature or slightly above, or reflux temperature.

Before carrying out any of these conversions, the effect, if any, on other substituents should be considered, and such reagents as are appropriate should be selected together with the adoption of such precautionary measures as are necessary. For example, O-alkylation and O-acylation may also produce N-alkylated and N-acylated products respectively unless the nitrogen atom(s) is (are) previously

protected. This may be conveniently achieved by carrying out the alkylation or acylation reaction in a strong acid, such as trifluoroacetic acid, which protonates, and thereby protects, the nitrogen atom(s).

Compounds of the formulae (XX) and (XXII) are novel intermediates and thus form an aspect of the present invention.

The compounds of formula (XVIII) are known or are preparable analogously to or routinely from known compounds. For example the compound, wherein v is O, $R_{13}$ and $R_{14}$ are each hydrogen, and $Q_2$ is bromo can be prepared by reacting a compound of formula (XXVI):

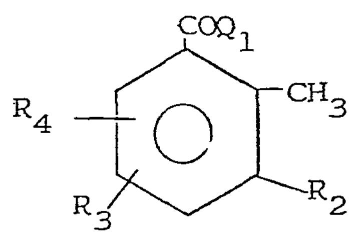

(XXVI)

wherein $R_2$, $R_3$, $R_4$ and $Q_1$ are as hereinbefore defined; with a brominating agent, such as N-bromosuccinimide.

The corresponding chloromethyl compound can be obtained similarly and this can then be base hydrolysed (then acylated) to give the corresponding compound of formula (XVIII) wherein $R_{13}$ and $R_{14}$ are both hydrogen and $Q_2$ is hydroxy (or acyloxy) respectively.

Alternatively, those compounds of formula (XVIII) wherein v is O or 1, and $Q_1$ and $Q_2$ together are O, can be prepared by known methods from phthalic or homophthalic acids respectively, for example by reflux with acetic anhydride.

BAD ORIGINAL

Compounds of formula (XVIII) wherein $R_2$, $R_3$ or $R_4$ is aminosulphonyl may be formed from the corresponding chlorosulphonyl derivatives with a suitable amine or ammonia.

It will be realised that in the compound of the formula (I) the -X-Y-linkage may have an  or  orientation with respect to the ring of the bicyclic moiety to which it is attached. A mixture of α and β isomers of the compound of the formula (I) may be synthesised non-stereospecifically and the desired isomer separated conventionally therefrom eg. by chromatography; or alternatively the α and β isomer may if desired be synthesised from the corresponding α or β form of the compound of the formula (XIX).

Synthesis from the corresponding α or β isomer of the compound of the formula (XIX) is in general preferred.

It will be appreciated that, when X is NH, Y is CO and n = O in the compounds of the formulae (I) or (XIX), epimerisation of the OC-ring linkage to the energetically more favourable orientation often takes place readily in the presence of acid or base. In such cases if the less favoured isomer is desired, it is preferred to stereospecifically synthesisethe isomer of the compound of the formula (I) under such conditions to avoid epimersation.

The  or  form of the compound of the formula (XIX) may if desired be prepared by known stereospecific processes, such as those leading to the α or β isomers of the compound of the formula (XIX) depicted in the Scheme and described in the Descriptions hereinafter.

Compounds of the formula (XIX) are known or are preparable conventionally.

SCHEME 1

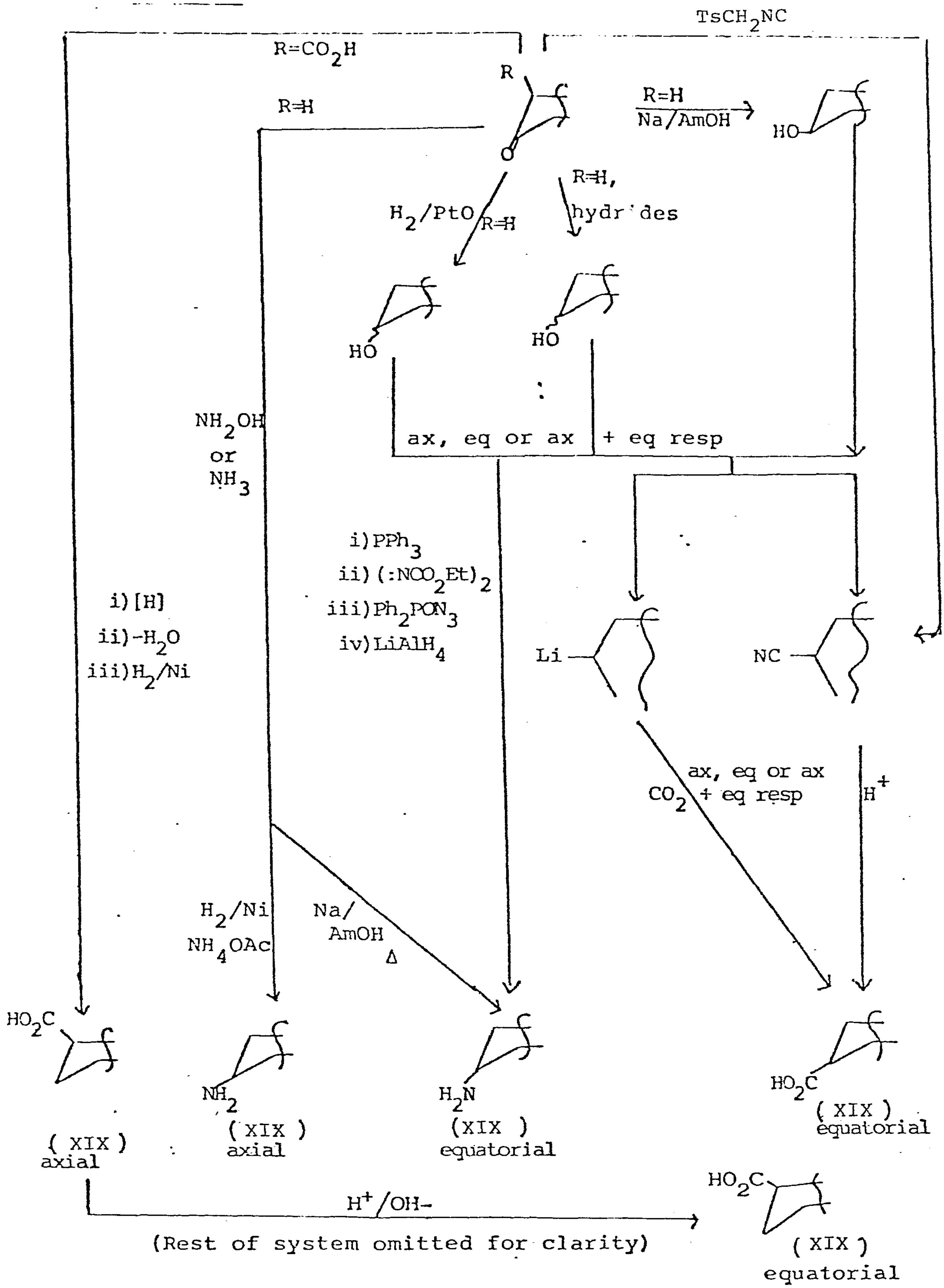

TsCH2NC
R=CO2H
R
R=H
R=H
Na/AmOH
HO
H2/PtO
R=H
R=H,
hydr'des
HO
HO
ax, eq or ax + eq resp
NH2OH
or
NH3
i) PPh3
ii) (:NCO2Et)2
iii) Ph2PON3
iv) LiAlH4
i) [H]
ii) -H2O
iii) H2/Ni
Li
NC
ax, eq or ax
CO2 + eq resp
H+
H2/Ni
NH4OAc
Na/
AmOH
Δ
HO2C
NH2
H2N
HO2C
(XIX)
axial
(XIX)
axial
(XIX)
equatorial
(XIX)
equatorial
H+/OH-
(Rest of system omitted for clarity)
HO2C
(XIX)
equatorial

Pharmaceutically acceptable salts, and N-oxides of the compounds of this invention may be formed conventionally. The salts may be formed for example by reaction of the base compound of formula (I) with a pharmaceutically acceptable inorganic or organic acid.

N-oxides of the nitrogen atom of the bicyclic ring system are produced by reaction of a compound of formula (I) with an organic peracid, such as m-chloroperbenzoic acid in, for example, a chlorinated hydrocarbon solvent at below ambient temperature.

Quaternary ammonium salts may be prepared by reaction of a compound of the present invention with the appropriate alkyl, aryl, aralkyl, chloride, bromide or iodide. This reaction may be carried out in a solvent, such as acetone, methanol, ethanol, dimethylformamide at ambient or elevated temperature with or without pressure.

The compounds of the present invention are dopamine antagonists and may generally be used in the treatment of emesis. Depending on their balance between peripheral and central action on the nervous system, they may also be used in the treatment of disorders relating to impaired gastro-intestinal motility, such as retarded gastric emptying, dyspepsia, flatulence, oesophagal reflux and peptic ulcer and/or in the treatment of disorders of the central nervous system, such as psychosis.

Compounds of the present invention which are of interest for their beneficial effect on gastric motility include the quaternary ammonium salts and N-oxides of compounds of the formula (I).

The invention also provides a pharmaceutical composition comprising a compound of the present invention, or a pharmaceutically acceptable salt or N-oxide thereof or a solvate of any of the foregoing and a pharmaceutically acceptable carrier.

Such compositions are prepared by admixture and are suitably adapted for oral or parenteral administration.

In order to obtain consistency of administration it is preferred that a composition of the invention is in the form of a unit-dose. Suitable unit dose forms may be in the form of tablets, capsules, oral liquid preparations, powders, granules, lozenges, reconstitutable powders, injectable and infusable solutions or suspensions or suppositories. Orally administerable compositions are preferred.

Tablets and capsules for oral administration usually contain conventional excipients such as binding agents, fillers, tabletting agents lubricants, disintegrants, and wetting agents. The tablets may be coated according to well known methods in the art. Oral liquid preparations are usually in the form of aqueous or oily suspensions, solutions, emulsions, syrups, or elixirs, or are presented as a dry product for reconstitution with water or other suitable vehicle before use. Such liquid preparations may contain conventional additives such as suspending agents, emulsifying agents, non-aqueous vehicles (which may include edible oils), preservatives, flavouring or colouring agents, and thickeners.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in the vehicle and filter sterilizing before filling into a suitable vial or ampoule and sealing. Advantageously, adjuvants such as a local anaesthetic, preservatives and buffering agents are also dissolved in the vehicle.

Parenteral suspensions are prepared in substantially the same manner except that the compound is suspended in the vehicle instead of being dissolved and sterilized by exposure to ethylene oxide before suspending in the sterile vehicle. Advantageously, a surfactant or wetting agent is included in the composition to facilitate uniform distribution of the compound of the invention.

The invention further provides a method of treatment or prophylaxis of emesis or disorders of the central nervous system in mammals, such as humans, which comprises the administration of an effective amount of a compound of the present invention, or a pharmaceutically acceptable acid addition salt thereof, or a solvate of either of the foregoing.

The invention further provides a method of treatment or prophylaxis of disorders related to impaired gastro-intestinal motility in mammals, such as humans, which comprises the administration of an effective amount of a compound of the present invention, or a pharmaceutically acceptable salt thereof, or N-oxide thereof, or a solvate of any of the foregoing.

An amount effective to treat the disorders hereinbefore described depends on the relative efficacies of the compounds of the invention, the nature and severity of the disorder being treated and the weight of the mammal. However, a unit dose will normally contain 0.1 to 20 mg for example 0.5 to 10 mg, of the compound of the invention. Unit doses will normally be administered more than once a day, for example 2, 3, 4, 5 or 6 times a day such that the total daily dose is normally in the range 0.01 to 10 mg/kg per day.

The invention provides a compound of the present invention or a pharmaceutically acceptable acid addition salt thereof, or a solvate of either of the foregoing for the treatment or prophylaxis of emesis, disorders related to impaired gastro-intestinal motility or of the central nervous system.

The invention also provides a quaternary salt or N-oxide of a compound of the present invention or a solvate thereof for the treatment or prophylaxis of disorders related to impaired gastro-intestinal motility.

The compound of the present invention also have the ability to potentiate the effect of conventional analgesics in migraine treatment when administered concurrently with the analgesic.

Accordingly, the present invention also provides a pharmaceutical composition comprising a compound of the present invention or a pharmaceutically acceptable salt or N-oxide thereof or a solvate of any of the foregoing, and an analgesic.

The compound of the present invention and the analgesic such as aspirin or paracetamol, are present in the composition in amounts generally similar to their usual effective dose.

The composition can be a combination product, for example a tablet or capsule containing both a compound of the invention and an analgesic for oral administration, or a twin pack comprising the two active ingredients made up for separate administration.

The invention accordingly provides a method of treatment of migraine in mammals including humans comprising the administration of an effective amount of a compound of the invention or a pharmaceutically acceptable salt or N-oxide thereof or a solvate of any of the foregoing and an analgesic.

The following examples illustrate the preparation of compounds of the invention and the following descriptions illustrate the preparation of intermediates.

0101641



DESCRIPTION 1

4-Bis(methanesulphonyl)amino-5-chloro-2-methoxy-N-[3β-(8-benzyl-8-azabicyclo(3.2.1)octyl)]benzamide. Compound D.1.

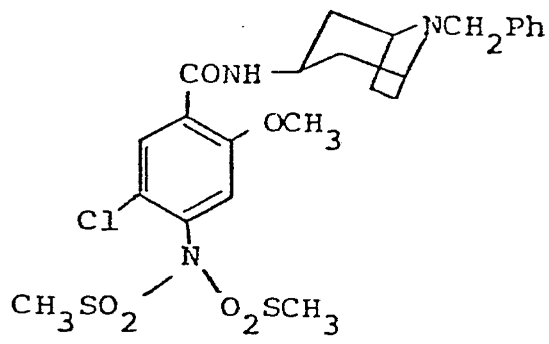

Methanesulphonyl chloride (0.97ml; 2eq) in dry methylene chloride (5ml) was added slowly to a stirred solution of 4-amino-5-chloro-2-methoxy-N-[3β-(8-benzyl-8-azabicyclo(3.2.1)octyl)]benzamide (2.5g) and triethylamine (1.82ml; 2.1eq) in dry methylene chloride (30ml), at room temperature. The mixture was heated at reflux for 3 hours. The cooled solution was washed with saturated sodium bicarbonate solution and with water then dried and evaporated, in vacuo, to give the title compound as a cream coloured solid (3.1g).

NMR ($CEC1_3$), τ 1.7 (1H, s, aromatic CH)
2.7 (5H, m, aromatic CH)
3.1 (1H, s, aromatic CH)
5.7 (1H, m, 3αH)
6.1 (3H, s, $OCH_3$)
6.5 (2H, s, $NCH_2Ph$)
6.6 (6H, s, 2 x $CH_3SO_2$-)
6.8 (2H, bs, bridgehead H's)
8.25 (8H, m, remaining H's)

Mass spectrum: observed 555.1233
calculated 555.1262

0101641

In a similar manner, using 4-amino-2-methoxy-N-[3β-(8-benzyl-8-azabicyclo (3.2.1)octyl)]benzamide, was prepared 4-bis(methanesulphonyl)amino-2-methoxy-N-[3β-(8-benzyl-8-azabicyclo(3.2.1)octyl)]benzamide. Compound (D2).

NMR ($CDCl_3$), τ 1.75 (1H, d, aromatic H)
2.3 to 3.1 (7H, m, aromatic H)
5.7 (1H, m, 3αH)
6.0 (3H, s, $OCH_3$)
6.4 (2H, s, $NC\underline{H}_2Ph$)
6.6 (6H, s, 2 x $C\underline{H}_3SO_2$-)
6.7 (2H, bs, bridgehead H's)
8.2 (8H, bm, remaining H's)

In a similar manner, using 4-amino-5-chloro-2-methoxy-N-[3β-{8-(3-methylbutyl)-8-azabicyclo(3.2.1)octyl}]benzamide, was prepared 4-bis(methanesulphonyl)amino-5-chloro-2-methoxy-N-[3β-{8-(3-methylbutyl)-8-azabicyclo(3.2.1)octyl}]benzamide  Compound D.3.

In a similar manner are prepared:

4-bis(methanesulphonyl)amino-5-chloro-2-methoxy-N-{3β-(8-cyclohexyl-8-azabicyclo{3.2.1}octyl)}benzamide, (D.4), and

4-bis(methanesulphonyl)amino-5-chloro-2-methoxy-N-{3β-(8-(2-thienyl)-8-azabicyclo{3.2.1}octyl)}benzamide, (D.5).

Description 2

4-Bis(methanesulphonyl)amino-2-methoxy-5-methylsulphonyl-N-[3β-(8-benzyl-8-azabicyclo(3.2.1)octyl)]benzamide.

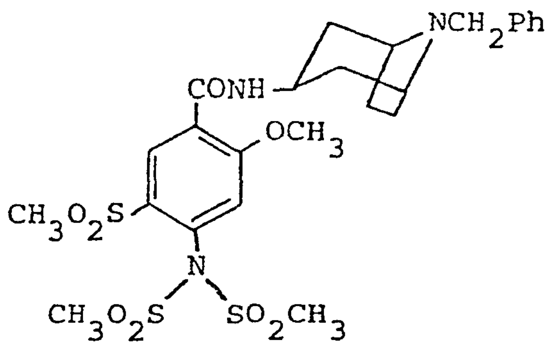

D. 5

Methane sulphonyl chloride (758mg; 3 equiv) in dry methylene chloride (5ml) was added slowly to a stirred ice-cold mixture of 4-amino-2-methoxy-5-methylsulphonyl-N-[3β-{8-benzyl-8-azabicyclo(3.2.1)octyl}]benzamide (1g - and triethylamine
(735mg; 3.3 equiv) in dry methylene chloride (50ml), under nitrogen. The resulting mixture was heated at reflux, under nitrogen, overnight.

The cooled solution was washed with saturated sodium bicarbonate solution and with water then dried and evaporated in vacuo to give a mixture of the title compound (≈ 70%) and the starting amine (≈ 30%). Trituration with methanol gave the title compound (753mg).

| | | |
|---|---|---|
| NMR ($CDCl_3$), τ | 1.13 | (1H, s, aromatic H) |
| | 2.65 | (5H, m, aromatic H's) |
| | 3.02 | (1H, s, aromatic H) |
| | 5.7 | (1H, bm, 3α-H) |
| | 5.9 | (3H, s, $OCH_3$) |
| | 6.45 | (8H, bs, $NC\underline{H}_2Ph$; 2 x $CH_3SO_2N$-) |
| | 6.7 | (2H, bs, bridgehead H's) |
| | 6.75 | (3H, s, $CH_3SO_2$-) |
| | 8.15 | (8H, m, remaining H's) |

In a similar manner, using 4-amino-3-hydroxy-2-methoxy-N-{3β-(8-benzyl-8-azabicyclo(3.2.1)octyl}benzamide is prepared 4-bis(methanesulphonyl)amino-3-methanesulphonyloxy-2-methoxy-N-{3β-(8-benzyl-8-azabicyclo(3.2.1)octyl}benzamide (D.7), and, using 3-amino-6-nitro-2-methoxy-N-{3β-(8-benzyl-8-azabicyclo(3.2.1)octyl}benzamide is prepared 3-bis(methanesulphonyl)amino-6-nitro-2-methoxy-N-{3β-(8-benzyl-8-azabicyclo(3.2.1)octyl}benzamide (D.8).

EXAMPLE 1

4-Methanesulphonamido-5-chloro-2-methoxy-N-[3β-(8-benzyl-8-azabicyclo[3.2.1]octyl)]benzamide, Compound (1).

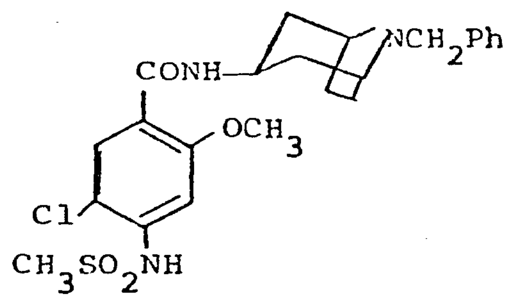

4-Bis(methanesulphonyl)amino-5-chloro-2-methoxy-N-[3β-(8-benzyl-8-azabicyclo(3.2.1)octyl)]benzamide (3g) was warmed at 50°C, for ½ hour, with 10% sodium hydroxide solution (40ml) and methanol (5ml). The resultant solution was cooled and acidified with conc. hydrochloric acid. The aqueous phase was neutralised with sodium bicarbonate solution, treated with solid sodium chloride, and then extracted with dichloromethane. The dichloromethane solution was dried ($Na_2SO_4$) and evaporated in vacuo to give a light brown foam (2.2g). This was chromatographed on silica gel (5:1) using 2% methanol in dichloromethane as eluant to give the title compound as a white solid (1.86g).

NMR[$(CD_3)_2SO$], τ 2.05 (1H, d, CONH)
2.3 (1H, s, aromatic H)
2.5 (5H, bm, aromatic H's)
2.8 (1H, s, aromatic H)
5.8 (1H, bm, 3αH)
6.1 (2H, s, $NC\underline{H}_2Ph$)
6.15 (3H, s, $OCH_3$)
6.55 (2H, bs, bridgehead H's)
6.9 (3H, s, $CH_3SO_2$-)
8.2 (8H, bm, remaining H's)

Mass spectrum: observed 477.1484
calculated 477.1486

In a similar manner, using 4-bis(methanesulphonyl)amino-2-methoxy-N-[3β-(8-benzyl-8-azabicyclo-(3.2.1)octyl)]benzamide (Compound D.2.) was prepared 4-methanesulphonamido-2-methoxy-N-[3β-(8-benzyl-8-azabicyclo(3.2.1)octyl)]benzamide. (Compound 2).

NMR [$(CD_3)_2SO$], τ 2.2 (1H, d, CONH)
2.2 to 3.2 (8H, bm, aromatic H's)
5.8 (1H, bm, 3αH)
6.1 (3H, s, $OCH_3$)
6.2 (2H, s, $NC\underline{H}_2Ph$)
6.6 (2H, bs, bridgehead H's)
6.9 (3H, s, $CH_3SO_2$-)
8.2 (8H, bm, remaining H's)

Mass spectrum: Observed 443.1906
Calculated 443.1876

In a similar manner, using 4-bis(methanesulphonyl) amino-5-chloro-2-methoxy-N-[3β-{8-(3-methylbutyl)-8-azabicyclo(3.2.1)octyl}]benzamide was prepared 5-chloro-4-methanesulphonamido-2-methoxy-N-[3β-{8-(3-methylbutyl)-8-azabicyclo (3.2.1)octyl}]benzamide (Compound 3).

| | | |
|---|---|---|
| NMR [$(CD_3)_2SO$] τ | 2.25 | (1H, d, CONH) |
| | 2.33 | (1H, s, aromatic H) |
| | 2.9 | (1H, s, aromatic H) |
| | 5.15 | (1H, b, $SO_2NH$) |
| | 5.85 | (1H, m, 3αH) |
| | 6.17 | (3H, s, $OCH_3$) |
| | 6.35 | (2H, bs, bridgehead H's) |
| | 7.15 | (3H, s, $SO_2CH_3$) |
| | 7.3 | (2H, m, $CH_2N$) |
| | 7.75 to 8.75 | (11H, m, aliphatic H's) |
| | 9.1 | (6H, d, $CH(\underline{CH_3})_2$ |

In a similar manner using (D4) and (D5) respectively are prepared:

4-amino-5-chloro-2-methoxy-N-[3β-{8-cyclohexyl-8-azabicyclo(3.2.1)octyl}]benzamide (4), and
4-amino-5-chloro-2-methoxy-N-[3β-{8-(2-thienyl)-8-azabicyclo(3.2.1)octyl}]benzamide (5).

EXAMPLE 2

4-Methanesulphonamido-2-methoxy-5-methylsulphonyl-N-[3β-{8-benzyl-8-azabicyclo(3.2.1)octyl}]benzamide Compound 6

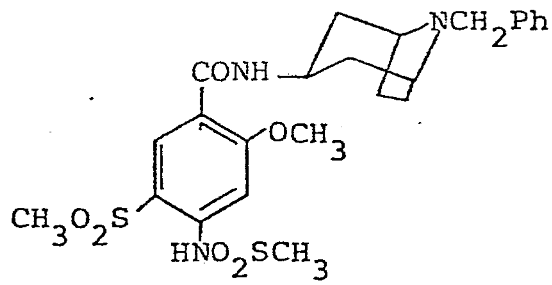

4-Bis(methanesulphonyl)amino-2-methoxy-5-methylsulphonyl-N-[3β-{8-benzyl-8-azabicyclo(3.2.1)octyl}]benzamide (750mg) was warmed at 50°, for 1hr, with 10% sodium hydroxide solution (12ml) and methanol (1.5ml). The resulting solution was cooled and dilute hydrochloric acid was added dropwise until the mixture was neutral. A brown gum and white crystals were deposited. The brown gum was re-crystallised from ethanol to give the title compound (197mg) as the hydrochloric salt.

NMR [$(CD_3)_2SO$], Temperature 66°C

τ
0.4 (1H, b, $CH_3SO_2\underline{NH}$)
1.8 (1H, s, aromatic H)
2.23 (1H, d, CONH)
2.35 (2H, m, aromatic H's)
2.57 (3H, m, aromatic H's)
2.83 (1H, s, aromatic H)
5.7 (1H, m, 3αH)
5.85 (2H, s, $CH_2Ph$)
6.08 (3H, s, $OCH_3$)
6.27 (2H, bs, bridgehead H's)
6.75 (3H, s, $SO_2CH_3$)
6.95 (3H, s, $SO_2CH_3$)
7.5 to 8.1 (8H, m, remaining protons)

In a similar manner, using (D.7), is prepared 4-methanesulphonamido-3-hydroxy-2-methoxy-N-{3β-(8-benzyl-8-azabicyclo(3.2.1)octyl}benzamide (7), and, using (D.8), 3-methanesulphonylamino-6-nitro-2-methoxy-N-{3β-(8-benzyl-8-azabicyclo(3.2.1)octyl}benzamide (8).

(8) is reduced using Raney nickel . to 3-methanesulphonylamino-6-amino-2-methoxy-N-{3β-(8-benzyl-8-azabicyclo(3.2.1)octyl}benzamide (9).

Example 3

5-Methanesulphonylamido-2-methoxy-3-methyl-N-[3α-{8-benzyl-8-azabicyclo(3.2.1)octyl}]benzamide (10)

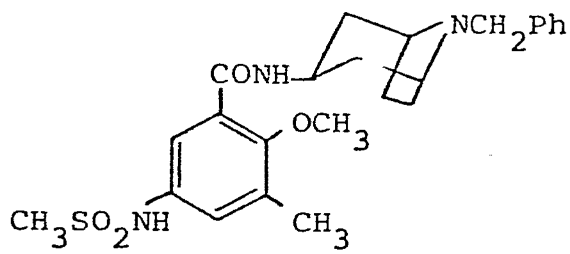

Methanesulphonylchloride (305mg; 2.44 equiv) in dry methylene chloride (5ml) was added slowly to an ice-cold mixture of 5-amino-2-methoxy-3-methyl-N-[3β-{8-benzyl-8-azabicyclo(3.2.1)octyl}]benzamide (500mg) and triethylamine (293mg; 2.64 equiv) in dry dichloromethane (10ml). The resulting mixture was stirred at room temperature, under nitrogen for 30 minutes. The methylene chloride solution was washed with saturated aqueous sodium bicarbonate and with water then dried and evaporated in vacuo. The residue was warmed at 50° for 30 minutes with 10% aqueous sodium hydroxide (9ml) and methanol (1ml). The cooled mixture was neutralised and the product was extracted with methylene chloride. The methylene chloride solution was dried and evaporated to give a white solid (500mg) which was chromatographed on silica gel (10:1) using methylene chloride then 2% methanol in methylene chloride as eluants. The title compound was obtained as a white solid (215mg).

NMR [$(CD_3)_2SO$]

| | | |
|---|---|---|
| τ | 0.45 | (1H, b, $SO_2NH$) |
| | 1.97 | (1H, d, CONH) |
| | 2.5 to 2.9 | (7H, m, aromatic H's) |
| | 5.85 | (1H, m, 3αH) |
| | 6.35 | (3H, s, $OCH_3$) |
| | 6.45 | (2H, s, $CH_2Ph$) |
| | 6.8 | (2H, m, bridgehead H's) |
| | 7.05 | (3H, s, $SO_2CH_3$) |

7.8 (3H, s, $CH_3$)
7.8 to 8 (8H, m, remaining H's)

Mass spectrum: observed 457.2027
calculated 457.2032

Pharmacological Data

Increase in intragastric pressure - peripheral dopamine blockade

Intragastric pressure changes were recorded from previously starved conscious but restrained rats using a saline filled catheter inserted into the lumen of the stomach *via* a permanent gastric fistula. The catheter was connected to a physiological pressure transducer and pressure changes recorded on a hot wire pen recorder. In each animal a pre-dose period of 40 minutes was allowed to obtain a measure of spontaneous activity. An index of activity was obtained by measuring the average height of pressure waves during 10 minute periods. Values for 4 such periods were obtained during assessment of spontaneous activity and for 40 minute period after administration of compound. Student's "T" test was applied to the difference in average values obtained for spontaneous and post compound activity.

Anti-emetic activity in the dog

Compounds were administered subcutaneously 30 minutes prior to administration of a standard dose of apomorphine HCl (0.1 mg/kg subcutaneously) and the vomiting response compared to that obtained when the same animals were dosed with apomorphine HCl and vehicle only.

Dopamine Receptor Blocking Activity in the Central Nervous System

Compounds were tested for inhibition of apomorphine induced climbing in the mouse. The test is based on that described by Protais, P., Constantin, J. and Schwartz J.C. (1976), Psychopharmacology, 50, 1.6.

Apomorphine 1mg/kg s.c. induces mice to climb the wall of a wire cage (inverted food hopper - 11 x 7.5 x 18cm high). Mice acclimatised in their home cages in groups of 5 are placed under the hoppers immediately after the injection of apomorphine 1mg/kg s.c. At 10, 20 and 30 minutes after injection, climbing behaviour is scored. The mice are observed for 30 seconds and scored according to the position they spend the majority of time in, score 0 - four paws on floor of cage; score 1 - fore paws only on walls; score 2 - all paws on wall of cage. The scores at all 3 times and for each mouse are summed and mice drug treated orally compared to mice receiving apomorphine only. A saline only treated group is also included and any score, generally >5% of maximum taken into account.

The results were as shown in Table 1.

Toxicity

No toxic effects were observed in any of the above tests.

TABLE 1

| Compound | IG Pressure (Basal) mg/kg s.c. | Anti-emetic mg/kg s.c. | CNS (Climbing) mg/kg s.c. |
|---|---|---|---|
| 1 | A at 1.0 | $ED_{50}$ 0.02 | $ED_{50}$ 0.2 |
| 2 | IA at 1.0 | $ED_{50}$ 0.1 | A at 10 |
| 6 | IA at 5.0 | A at 0.1 | IA at 50 |
| 10 | A at 1.0 | A at 0.1 | $ED_{50}$ 9.3 |

A = active IA = inactive

## Claims

1. A compound of formula (I), or a pharmaceutically acceptable salt, or N-oxide thereof, or a solvate of any of the foregoing:

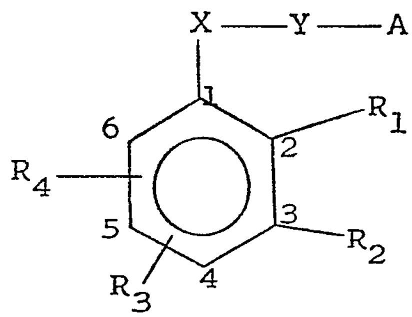

(I)

wherein:

one of X and Y is CO and the other is $NR_9$, $R_9$ being hydrogen or, with $R_1$; as defined below, when X is CO and Y is $NR_9$;

A is a group

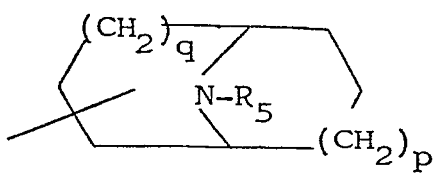

(II)

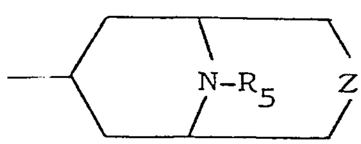

(III)

or

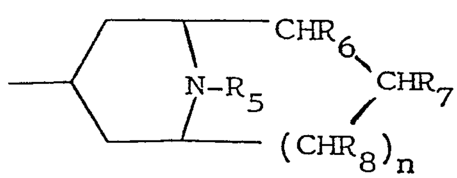

(IV)

where

p and q each independently are 0 to 2;

Z is O or S;

n is 0 or 1; and

one of $R_6$ and $R_7$ when n=0 is $C_{1-4}$ alkoxy, $C_{1-4}$ alkoxycarbonyl, hydroxy or $C_{1-4}$ alkyl optionally substituted by hydroxy, $C_{1-4}$ alkoxy or $C_{1-4}$ acyloxy, and the other is hydrogen or $C_{1-4}$ alkyl or one of $R_6$, $R_7$ and $R_8$ when n=1 is $C_{1-4}$ alkyl and the other two are the same or different and are hydrogen or $C_{1-4}$alkyl.

$R_5$ is $C_{1-7}$ alkyl, $-(CH_2)_sR_{10}$, s being 0 to 2 and $R_{10}$ being $C_{3-8}$ cycloalkyl, $-(CH_2)_tR_{11}$, t being 1 or 2 and $R_{11}$ being thienyl or phenyl optionally substituted by one or two substituents selected from $C_{1-4}$ alkoxy, trifluoromethyl, halogen, nitro, carboxy, esterified carboxy and $C_{1-4}$ alkyl optionally substituted by hydroxy, $C_{1-4}$ alkoxy, carboxy, esterified carboxy or <u>in vivo</u> hydrolysable acyloxy:

and either

$R_1$ is $C_{1-6}$ alkoxy, $C_{1-6}$ alkylthio or, together with $R_9$ when Y is $NR_9$, $C_{1-2}$ alkylene,

one of $R_2$, $R_3$ and $R_4$ is $R_{15}SO_2NR_{20}$ where $R_{15}$ is $C_{1-6}$ alkyl, or amino optionally substituted by one or two groups selected from $C_{1-6}$ alkyl, $C_{3-8}$ cycloalkyl, $C_{3-8}$ cycloalkyl $C_{1-4}$ alkyl, phenyl or phenyl $C_{1-4}$ alkyl groups, any of which phenyl groups may be substituted by one or more groups selected from halogen, trifluoromethyl, $C_{1-6}$ alkyl, $C_{1-6}$ alkoxy and nitro, or optionally substituted by $C_{4-5}$ polymethylene, and $R_{20}$ is hydrogen or $C_{1-4}$alkyl, and the other two of $R_2$,$R_3$ and $R_4$ together are $C_{1-2}$ alkylenedioxy, or the other two are the same or different and are selected from hydrogen, halogen trifluoromethyl, $C_{1-6}$ alkyl, $C_{1-6}$ alkoxy, $C_{1-6}$ alkylthio, $C_{1-7}$ acyl, carboxylic $C_{1-7}$ acylamino, $C_{1-6}$ alkylsulphonyl, $C_{1-6}$ alkylsulphinyl, hydroxy, nitro or amino, aminocarbonyl or aminosulphonyl optionally N-substituted by one or two groups selected from $C_{1-6}$ alkyl, $C_{3-8}$ cycloalkyl, $C_{3-8}$ cycloalkyl $C_{1-4}$ alkyl, phenyl or phenyl $C_{1-4}$ alkyl groups or optionally N-disubstituted by $C_{4-5}$ polymethylene,

or

$R_1$ and $R_2$ together are $C_{1-2}$ alkylenedioxy;

one of $R_3$ and $R_4$ is $R_{15}SO_2NR_{20}$ as defined, and the other is selected from the groups defined hereinbefore for $R_3$ and $R_4$.

2. A compound according to claim 1 of formula (VI):

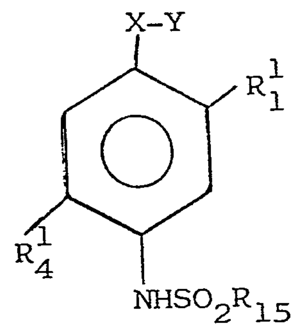

(VI)

wherein $R^1_1$ is $C_{1-6}$ alkoxy;
$R^1_4$ is H,Cl or $C_{1-6}$ alkylsulphonyl and the remaining variables are as defined in claim 1.

3. A compound according to claim 2 of formula (VIII):

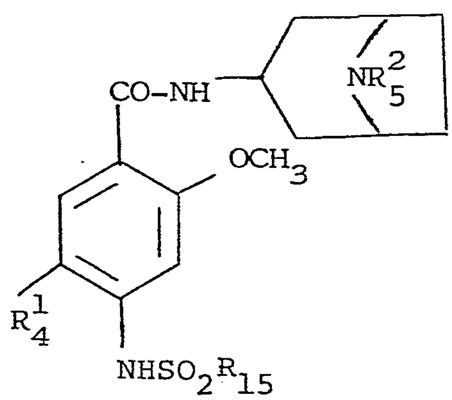

(VIII)

wherein

$R^2_5$ is thienylmethyl or $-(CH_2)_tR_{11}$, t being 1 or 2, and $R_{11}$ being phenyl optionally substituted by one or two substituents selected from $C_{1-4}$ alkoxy, trifluoromethyl, halogen, carboxy, esterified carboxy, and $C_{1-4}$ alkyl optionally substituted by hydroxy, $C_{1-4}$ alkoxy, carboxy, esterified carboxy or <u>in vivo</u> hydrolysable acyloxy, and the remaining variables are as defined in claim 2.

0101641

4. A compound according to claim 3 which is

4-methanesulphonamido-5-chloro-2-methoxy-N-[3β-(8-benzyl-8-azabicyclo(3.2.1)octyl)]benzamide.

4-methanesulphonamido-2-methoxy-N-[3β-(8-benzyl-8-azabicyclo(3.2.1)octyl)]benzamide,

4-methanesulphonamido-5-methanesulphonyl-2-methoxy-N-[3β-(8-benzyl-8-azabicyclo(3.2.1)octyl)]benzamide,

4-methanesulphonamido-5-chloro-2-methoxy-N-{3β-[8-(3-methylbutyl)-8-azabicyclo(3.2.1)octyl]}benzamide,

5-methanesulphonamido-2-methoxy-3-methyl-N-{3β-[8-benzyl-8-azabicyclo(3.2.1)octyl]}benzamide,

or a pharmaceutically acceptable salt, or N-oxide thereof, or a solvate of any of the foregoing.

5. A process for the preparation of a compound according to claim 1 which process comprise reacting a compound of formula (XX):

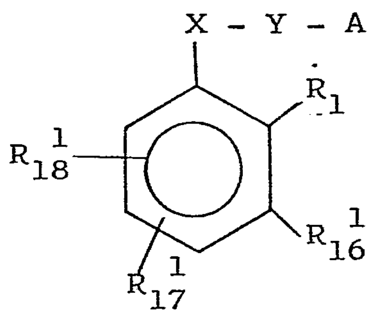

(XX)

wherein X, Y and A are as defined in claim 1,

one of $R_{16}^{1}$, $R_{17}^{1}$ and $R_{18}^{1}$ is amino or $C_{1-6}$ alkylamino and the other two are as defined for $R_2$, $R_3$ and $R_4$ in claim 1 or any group convertible thereto.

but none is $R_{15}SO_2NR_{20}$ as defined in claim 1, with a compound of formula (XXI):

$$R_{15}SO_2Q_4 \qquad (XXI)$$

as defined in claim 1; and as necessary thereafter selectively monodeacylating the resultant compound.

6. A pharmaceutical composition comprising a compound according to claim 1 or a pharmaceutically acceptable salt or N-oxide thereof or a solvate of any of the foregoing and a pharmaceutically acceptable carrier.

7. A compound of formula (XX) as defined in claim 5.

8. A compound according to claim 1 or a pharmaceutically acceptable acid addition salt thereof, or a solvate of either of the foregoing for the treatment or prophylaxis of emesis, or disorders related to impaired gastro-intestinal motility or of the central nervous system.

9. A compound according to claim 1 or a pharmaceutically acceptable salt thereof, or N-oxide thereof or a solvate of any of the foregoing for the treatment or prophylaxis of disorders related to impaired gastro-intestinal motility.